# EUROPÄISCHE PATENTSCHRIFT

(11) **EP 0 312 922 B1**
(45) Veröffentlichungstag und Bekanntmachung des Hinweises auf die Patenterteilung: **02.06.1993**
(21) Anmeldenummer: 88117081.5
(22) Anmeldetag: 14.10.1988
(51) Int. Cl.: C07C 315/02, C07C 317/14, C09B 62/507

(54) **Oxethylsulfonyl-nitro- bzw. -amino-benzoesäuren und Verfahren zu ihrer Herstellung**
Oxethylsulfonyl-nitro- or -amino-benzoic acids and process for their preparation
Acides oxéthylsulfonyl-nitro respectivement amino-benzoiques et procédé de leur préparation

(30) Priorität: 17.10.1987 DE 3735268
(43) Veröffentlichungstag der Anmeldung: 26.04.1989
(73) Patentinhaber: HOECHST AKTIENGESELLSCHAFT, 65926 Frankfurt am Main (DE)
(72) Erfinder: Papenfuhs, Theodor, Dr., D-6000 Frankfurt am Main 50 (DE)

(56) Entgegenhaltungen:
- EP-A- 0 171 611
- DE-A- 938 143
- DE-A- 3 026 808
- GB-A- 1 361 523

## Beschreibung

Die vorliegende Erfindung betrifft neue Oxethylsulfonyl-nitro- und teilweise neue Oxethylsulfonyl-aminobenzoesäuren und Verfahren zu ihrer Herstellung, indem man Halogen-nitrobenzoesäuren mit Mercaptoethanol zu den entsprechenden Oxethylmercapto-nitrobenzoesäuren umsetzt, diese zu den entsprechenden Oxethylsulfonyl-nitrobenzoesäuren oxidiert und letztere ggfs. zu den entsprechenden Oxethylsulfonyl-aminobenzoesäuren reduziert. Oxethylsulfonyl-aminobenzoesäuren sind wertvolle Diazokomponenten zur Herstellung faserreaktiver Azofarbstoffe (DE-PS 938 143 und 938 145), was nicht nur für die bisher bekannten, sondern auch für die neuen Oxethylsulfonyl-aminobenzoesäuren gilt, deren Vorstufen die entsprechenden neuen Oxethylsulfonyl-nitrobenzoesäuren sind.

Die bisher bekannten und für den genannten Zweck eingesetzten 4- bzw. 5-Oxethylsulfonyl-anthranilsäuren (DE-PS 938 143, DE-OS 2 222 096/3 145 571/3 026 808) sind nur auf verfahrenstechnisch, ökologisch und wirtschaftlich unbefriedigenden Wegen aus den entsprechenden Acylanthranilsäure-sulfonsäuren zugänglich, für deren Herstellung ebenfalls nur technisch aufwendige mehrstufige Verfahren bekannt sind.

Es bestand daher ein erhebliches Interesse, technisch günstigere Herstellungsverfahren für die vorstehend genannten bekannten Oxethylsulfonyl-anthranilsäuren und die neuen Oxethylsulfonyl-aminobenzoesäuren (mit abweichendem Substitutionsmuster) zur Verfügung zu stellen. Die dadurch gestellte Aufgabe wird durch die vorliegende Erfindung gelöst.

Es wurde nämlich überraschenderweise gefunden, daß man die neuen Oxethylsulfonyl-nitrobenzoesäuren der allgemeinen Formel (1a) 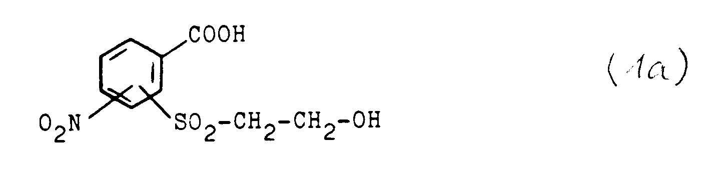
in welcher die Nitro- und Oxethylsulfonylgruppe in ortho- oder para-Stellung zueinander stehen, in vorteilhafter Weise herstellen kann, indem man 1 Mol einer Halogen-nitrobenzoesäure der allgemeinen Formel (2) 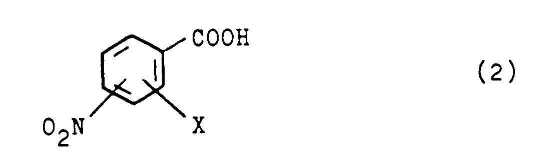
in welcher X ein Fluor-, Chlor-, Brom- oder Jodatom, vorzugsweise ein Chlor- oder Bromatom, bedeutet, und die Nitrogruppe und das Halogenatom in ortho- oder para-Stellung zueinander stehen, mit mindestens 1 Mol, vorzugsweise 1,1 bis 1,8 Mol, besonders bevorzugt 1,25 bis 1,6 Mol Mercaptoethanol in wäßriger Lösung oder Suspension oder in einem organischen Lösungsmittel in Gegenwart eines säurebindenden Mittels bei Temperaturen von 20 bis 100°C, vorzugsweise 35 bis 60°C, in die entsprechenden Oxethylmercapto-nitrobenzoesäuren der allgemeinen Formel (3) 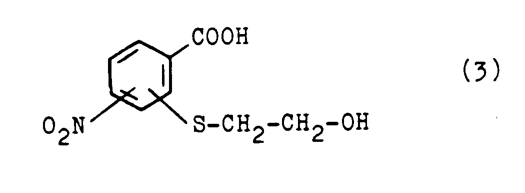
überführt, diese in an sich bekannter Weise zu den entsprechenden Oxethylsulfonyl-nitrobenzoesäuren der genannten Formel (1a) oxidiert und ggfs. letztere in an sich bekannter Weise zu den entsprechenden Oxethylsulfonyl-aminobenzoesäuren der allgemeinen Formel (4) 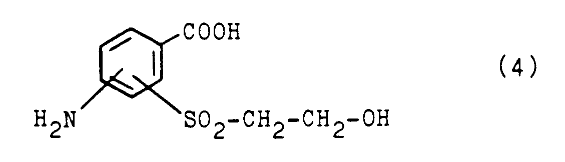
in welcher die Amino- und Oxethylsulfonylgruppe in ortho-oder para-Stellung zueinander stehen, reduziert.

Als Ausgangsverbindungen der genannten allgemeinen Formel (2) seien beispielsweise folgende genannt: 4-Chlor-3-nitro-benzoesäure, 2-Chlor-5-nitrobenzoesäure, 5-Chlor-2-nitrobenzoesäure, 3-Chlor-4-nitrobenzoesäure, 2-Chlor-3-nitrobenzoesäure und 3-Chlor-2-nitrobenzoesäure.

Die Umsetzung der Verbindungen der genannten allgemeinen Formel (2) mit Mercaptoethanol erfolgt bevorzugt in wäßrigem Medium in Abwesenheit eines organischen oder sonstigen Lösungsmittels. Eine Umsetzung in organischem Lösungsmittel kann beispielsweise in Dimethylformamid, Dimethylsulfoxid, N-Methylpyrrolidon oder Sulfolan erfolgen. Wird die Umsetzung (Kondensation) in einem der vorstehend genannten organischen Lösungsmittel vorgenommen, so kann zur ggf. gewünschten Isolierung der Verbindungen der allgemeinen Formel (3) ein vorheriges Verdünnen der Reaktionsmischung mit Wasser erforderlich sein.

Die Umsetzung des erfindungsgemäßen Verfahrens wird im einzelnen wie folgt vorgenommen:
Eine Halogennitrobenzoesäure gemäß Formel (2), vorzugsweise eine Chlor- oder Bromnitrobenzoesäure, wird in Wasser oder einem der beispielsweise genannten Lösungsmittel mit der berechneten Menge eines Alkalimetallhydroxids oder -carbonats, vorzugsweise eines Alkalimetallcarbonats, besonders bevorzugt des Kaliumcarbonats, neutralisiert. Anschließend wird das Mercaptoethanol in dem weiter oben genannten Molverhältnis zugesetzt und bei den weiter oben genannten Temperaturen innerhalb mehrerer Stunden (1 bis 5, vorzugsweise 2 bis 4 Stunden) eine dem Mercaptoethanol zumindest äquivalente Menge eines Alkalihydroxids oder -carbonats gleichmäßig eingetragen. Ein geringer Überschuß (etwa 5 bis 30 %) ist der Umsetzung nicht abträglich und daher im Prinzip statthaft, erhöht aber in der Regel nur den Salzanfall, ohne die Ausbeute oder Reinheit der gewünschten Oxethylmercapto-nitrobenzoesäuren der Formel (3) wesentlich zu beeinflussen. Nach erfolgter Zugabe wird bis zur Beendigung der Kondensation (Kontrolle durch Dünnschicht- oder Flüssigchromatographie) bei o.g. Temperatur nachgerührt, anschließend durch Zugabe von Mineralsäure (Salz-, Schwefel-oder Phosphorsäure) ein pH-Wert < 7,0, vozugsweise 1 bis 5, eingestellt, wobei die gebildete Oxethylmercapto-nitrobenzoesäure kristallin ausfällt und ggf. durch Filtration oder Zentrifugieren isoliert werden kann.

Was die beschriebene, erfindungsgemäße Umsetzung (Kondensation) der Verbindungen der allgemeinen Formel (2) mit Mercaptoethanol anbelangt, so ist es als überraschend anzusehen, daß die Umsetzung einheitlich und damit in hohen Ausbeuten durchführbar ist. Es war vielmehr zu erwarten, daß ein reduktiver Angriff der Nitrogruppe durch das Mercaptoethanol im alkalischen Medium des erfindungsgemäßen Verfahrens dem gewünschten Chloraustausch den Rang abläuft, dies umso mehr, als unter erfindungsgemäßen Reaktionsbedingungen das Halogennitrobenzoesäure-Substrat nicht als ggf. aktivierend wirkende freie Säure, sondern ausnahmlos als desaktiviertes Carboxylat-Anion der allgemeinen Formel (5) 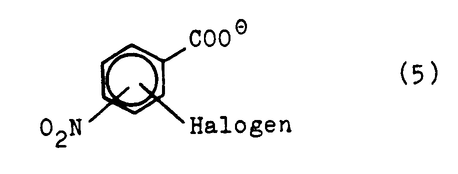
vorliegt, dessen gute Wasserlöslichkeit bei Durchführung der Umsetzung im wäßrigen Medium ebenfalls der konkurrierenden Reduktion der Nitrogruppe Vorschub leisten sollte. Demgegenüber war bei Durchführung der Umsetzung im Lösungsmittel zu erwarten, daß wegen der Schwerlöslichkeit der Ausgangssalze der vorstehend genannten Formel (5) die beabsichtigte Austauschreaktion nur stark verzögert ablaufen würde und damit Nebenreaktionen begünstigt werden würden.

Es war daher umso überraschender, daß die gewünschte Umsetzung von Halogennitrobenzoesäuren der genannten Formel (2) mit Mercaptoethanol in Gegenwart eines Säurebinders bei geschickter Wahl der Reaktionsbedingungen bei allen Substraten der Formel (2) so geführt werden kann, daß in hohen Ausbeuten die neuen Oxethylmercapto-nitrobenzoesäuren der Formel (3) resultieren, die dann durch Oxidation der Sulfidbrücke in die ebenfalls neuen Oxethylsulfonyl-nitrobenzoesäuren der Formel (1a) überführt und diese durch Reduktion zu teils neuen, teils bekannten Oxethylsulfonyl-aminobenzoesäuren der Formel (4) umgewandelt werden können.

Zur sich an die Kondensation anschließenden Oxidation ist in der Regel eine Isolierung der Oxethylmercapto-nitrobenzoesäure nicht erforderlich. Die Oxidation kann vielmehr besonders vorteilhaft direkt in der angefallenen Kondensationsmischung erfolgen, d.h. im Eintopf mit der Austauschreaktion durchgeführt werden.

Im einzelnen wird dabei zweckmäßigerweise wie folgt verfahren: Eine Mischung aus isolierter Oxethylmercapto-nitrobenzoesäure und der 3- bis 10-fachen, vorzugsweise 4- bis 6-fachen Menge Wasser, oder bevorzugt die angefallene Kondensationsmischung, wird mit Mineralsäure auf einen pH-Wert < 7, vorzugsweise 1 bis 6, eingestellt und auf Temperaturen von etwa 30 bis etwa 90°C, vorzugsweise etwa 45 bis etwa 75°C erwärmt. Man setzt als Katalysator eine Verbindung des 6-wertigen Wolframs (Natriumwolframat oder Wolframtrioxid) in einer Menge von 1 bis 10 Teilen, vorzugsweise 2 bis 4 Teilen pro Mol zu oxidierender Verbindung (Formel (3)) zu und tropft unter Rühren in 30 bis 240, vorzugsweise 60 bis 120 Minuten mindestens 2 Mol Wasserstoffperoxid (in Form einer wäßrigen, 20 bis 85 %igen, vorzugsweise 30 bis 50 %igen wäßrigen Lösung) pro Mol Oxethylmercapto-nitrobenzoesäure (oder beim Eintopfverfahren pro Mol eingesetztes Mercaptoethanol) zu (ein Überschuß von 5 bis 30 Molprozent ist besonders vorteilhaft) und führt die Oxidation durch 3- bis 10-stündiges, vorzugsweise 4- bis 6-stündiges Nachrühren bei Temperaturen von 60 bis 120°C, vorzugsweise 85 bis 100°C, zu Ende.

Dieses Oxidationsverfahren ist, insbesondere aus ökologischen Gründen, besonders bevorzugt. Es können aber auch andere literaturbekannte Oxidationsverfahren, beispielsweise mittels Halogen im sauren oder Hypohalogenit im alkalischen Medium, zur Anwendung kommen, wobei aber in der Regel nicht die Oxethylsulfonyl-, sondern die Chlorethylsulfonyl-nitrobenzoesäuren resultieren und erst in einer nachgeschalteten Verseifungsstufe in erstere umgewandelt werden müssen. Diese Oxidationsvarianten sind daher nicht bevorzugt.

Die aus der Oxidation, ggf. nach Verdünnen mit Wasser (falls eine lösungsmittelhaltige Kondensationsmischung eingesetzt wurde), beim Abkühlen auf -5° bis +10°C ausgefallene Oxethylsulfonyl-nitrobenzoesäure der Formel (4) wird durch Filtration oder Zentrifugieren isoliert und mit Wasser neutralgewaschen. Ein Trocknen erübrigt sich in der Regel, da die abschließende Reduktion im wäßrigen Medium erfolgt. Dabei kann sowohl eine Reduktion mit Eisen (Béchamp-Reduktion) als auch, besonders vorteilhaft, eine Hydrierung mit katalytisch aktiviertem Wasserstoff zur Anwendung kommen. Als Katalysatoren können sowohl handelsübliche Nickelkontakte (Raney-Nickel oder Nickel-Trägerkatalysatoren), als auch käufliche Edelmetallkatalysatoren, wie z.B. Palladium oder Platin auf inerten Trägern, vorzugsweise auf Aktivkohlen hoher spezifischer Oberfläche, eingesetzt werden.

Die Reduktion verläuft bei erhöhten Temperaturen (etwa 70 bis etwa 120°C, vorzugsweise etwa 80 bis etwa 100°C) in wäßriger Lösung oder Suspension in wenigen Stunden (1 bis 5 Stunden, vorzugsweise 1,5 bis 3 Stunden) quantitativ und liefert bei den angegebenen Reduktionstemperaturen wäßrige Lösungen der Zielverbindungen (Oxethylsulfonyl-aminobenzoesäuren der allgemeinen Formel (4)), aus denen die suspendierten Eisenoxidhydrate (im Falle der Béchamp-Reduktion) bzw. die Hydrierkatalysatoren durch Klärfiltration vorteilhaft abtrennbar sind.

Aus dem Klärfiltrat können die Oxethylsulfonyl-aminobenzoesäuren der allgemeinen Formel (4), ggf. nach Konzentrierung durch Abdestillieren von Wasser im Vakuum, durch Kühlen auf Temperaturen von -5 bis +20°C und/oder Aussalzen mit beispielsweise Natriumchlorid oder -sulfat ausgefällt und durch Filtration oder Zentrifugieren isoliert werden.

Die Ausbeuten und Selektivitäten sind in allen Stufen überraschend hoch und erreichen in den meisten Fällen Werte nahe der Theorie.

Die nachfolgenden Beispiele sollen die Erfindung näher erläutern, ohne sie darauf zu beschränken. Die angegebenen Teile sind Gewichtsteile.

### Beispiel 1

Eine Mischung aus 100,75 Teilen 6-Chlor-3-nitrobenzoesäure und 150 Teilen Wasser wird in 20 Minuten unter Rühren mit 105 Teilen Kaliumcarbonat versetzt, wobei sich eine Endtemperatur von ca. 40°C einstellt.

Man heizt auf 75°C und tropft dann in 60 Minuten 69 Teile Mercaptoethanol zu, rührt ca. 4 Stunden bei 90°C nach, bis im Dünnschichtchromatogramm kein Ausgangsprodukt mehr nachweisbar ist.

Anschließend kühlt man auf 15 bis 20°C ab, stellt mit 30%iger Salzsäure (ca. 175 Teile) einen pH-Wert von 1 ein, rührt 60 Minuten bei 15 bis 20°C nach, saugt den ausgefallenen, leicht gelb gefärbten Niederschlag ab, wäscht ihn mit Kaltwasser chloridfrei und trocknet im Vakuum bei 80°C.

Man erhält 117 Teile 2-Oxethylmercapto-5-nitrobenzoesäure der Formel 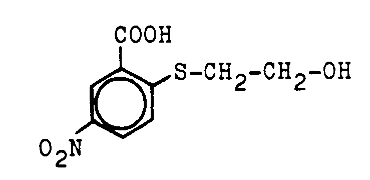
vom Schmelzpunkt 189 bis 191°C und einem Reingehalt (HPLC) von 99,1 %.

| | | | |
|---|---|---|---|
| Analyse: | S: | 13,2/13,0 % | (ber. 13,17 %) |
| | N: | 5,7/5,7 % | (ber. 5,76 %) |
| | Cl: | < 0,3 % | (ber. 0,0 %) |

Ersetzt man das Kaliumcarbonat durch aliquote Mengen Kalium- oder Natriumhydroxid (in Form von 30 bis 50%igen wäßrigen Lösungen) und arbeitet sonst in der angegebenen Weise, so erhält man bei Einsatz von Kaliumhydroxid ein vergleichbares Ergebnis, bei Einsatz von Natriumhydroxid selbst nach Verdoppelung der Reaktionszeit noch keine vollständige Umsetzung (Ausbeute: 104 Teile 2-Oxethylmercaptobenzoesäure vom Schmelzpunkt 181 bis 184°C, Reingehalt 96,2 %).

### Beispiele 2 bis 5

Ersetzt man im Beispiel 1 die 6-Chlor-3-nitrobenzoesäure durch die in Tabelle 1 angegebenen isomeren Chlornitrobenzoesäuren und arbeitet in der angegebenen Weise, so erhält man die entsprechenden Oxethylmercapto-nitrobenzoesäuren in den ebenfalls in Tabelle 1 aufgeführten Ausbeuten und Qualitäten (Schmelzpunkt/Reingehalt).

**Tabelle 1**

| Bsp. | Ausgangsprodukt | Zielprodukt | | | |
|---|---|---|---|---|---|
| | | Struktur | Schmelzpunkt | Ausbeute | RG (HPLC) |
| 2 | 5-Chlor-2-nitrobenzoesäure | 5-Oxethylmercapto-2-nitrobenzoesäure | 130 - 132°C | 98,8 % | 97,7 % |
| 3 | 4-Chlor-3-nitrobenzoesäure | 4-Oxethylmercapto-3-nitrobenzoesäure | 179 - 182°C | 97,1 % | 98,4 % |
| 4 | 3-Chlor-4-nitrobenzoesäure | 3-Oxethylmercapto-4-nitrobenzoesäure | 183 - 185°C | 95,0 % | 99,2 % |
| 5 | 3-Chlor-2-nitrobenzoesäure | 3-Oxethylmercapto-2-nitrobenzoesäure | 138 - 140°C | 93,8 % | 98,0 % |
| Die Analysen der Zielprodukte bestätigen die angegebenen Strukturen. | | | | | |

### Beispiel 6

In 50 Teile N-Methylpyrrolidon werden unter Rühren nacheinander 20,15 Teile 2-Chlor-3-nitrobenzoesäure und 20,5 Teile Kaliumcarbonat eingetragen. Man heizt auf 60°C und tropft dann in 30 Minuten 27,6 Teile 50%iges wäßriges Mercaptoethanol zu.

Nach Heizen auf 80 bis 90°C wird so lange nachgerührt, bis im Dünnschichtchromatogramm kein Ausgangsprodukt mehr nachweisbar ist (ca. 20 Stunden), setzt dann soviel Wasser zu, daß die ausgefallenen Salze bei 90°C im Lösung gehen, fügt 1 Teil Aktivkohle zu und klärt das Reaktionsgemisch über eine vorgeheizte Nutsche.

Das Klärfiltrat wird mit ca. 30 Teilen 30%iger Salzsäure auf pH 1 gestellt und auf 0 bis 5°C abgekühlt. Das sich zunächst ölig abscheidende Reaktionsprodukt kristallisiert bei mehrstündigem Nachrühren bei 0 bis 5°C langsam durch, wird abgesaugt, mit Eiswasser chlorid- und lösungsmittelfrei gewaschen und im Vakuum bei 60°C getrocknet. Man erhält 20,0 Teile 2-Oxethylmercapto-3-nitrobenzoesäure der Formel 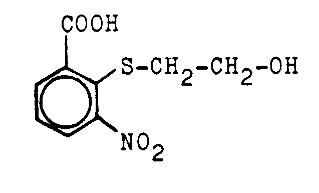
vom Schmelzpunkt 91 bis 93°C und einem Reingehalt (HPLC) von 99,4 %.

Ersetzt man das N-Methylpyrrolidon durch Dimethylacetamid und die 30%ige Salzsäure durch aliquote Mengen 20%ige Schwefel- oder Phosphorsäure und arbeitet sonst in der angegebenen Weise, so erhält man ein vergleichbares Ergebnis.

### Beispiel 7

Eine gerührte Suspension aus 486 Teilen 4-Oxethylmercapto-3-nitrobenzoesäure und 2500 Teilen Wasser wird mit ca. 100 Teilen Eisessig auf pH 1 gestellt, dann mit 9 Teilen Natriumwolframat-dihydrat versetzt und auf 75°C erwärmt. Man tropft nun, anfangs langsam und ohne Heizung, nach Abklingen der zunächst exothermen Reaktion zügig unter zusätzlichen Heizen 408 Teile 35%iges wäßriges Wasserstoffperoxid so zu, daß die Innentemperatur zwischen 70 und 80°C gehalten werden kann, heizt dann auf 95°C und rührt 4 bis 5 Stunden bei dieser Temperatur nach, bis im Dünnschichtchromatogramm das Ausgangsprodukt und das intermediär gebildete Sulfoxid nicht mehr sichtbar sind. Anschließend wird auf 0 bis 5°C abgekühlt, 2 Stunden nachgerührt und der ausgefallene farblose Niederschlag auf einer Nutsche isoliert. Nach Neutralwaschen mit Eiswasser und Trocknen im Vakuum bei 80°C erhält man 525 Teile 4-Oxethylsulfonyl-3-nitrobenzoesäure der Formel 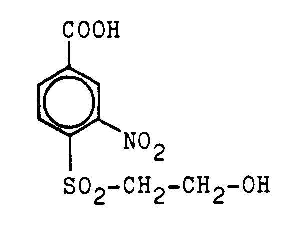
vom Schmelzpunkt 191 bis 193°C und einem Reingehalt (HPLC) von 98,8 %.

| | | | |
|---|---|---|---|
| Analyse: | S: | 11,6/11,5 % | (ber. 11,64 %) |
| | N: | 5,0/5,1 % | (ber. 5,09 %) |

Ersetzt man das Natriumwolframat-dihydrat durch aliquote Teile Wolframtrioxid und arbeitet sonst in der angegebenen Weise, so erhält man ein identisches Ergebnis.

Wird zum Einstellen des pH-Wertes an Stelle von Eisessig eine entsprechende Menge einer Mineralsäure (Salz- oder Schwefelsäure) eingesetzt und sonst analog verfahren, so erhält man die 4-Oxethylsulfonyl-3-nitrobenzoesäure in vergleichbarer Ausbeute und Qualität.

### Beispiele 8 bis 12

Ersetzt man im Beispiel 7 die 4-Oxethylmercapto-3-nitrobenzoesäure durch die in Tabelle 2 aufgeführten isomeren Ausgangsprodukte und arbeitet sonst in der angegebenen Weise, so erhält man die entsprechenden Oxethylsulfonyl-nitrobenzoesäuren in den ebenfalls in Tabelle 2 angegebenen Ausbeuten und Qualitäten (Schmelzpunkt/Reingehalt).

**Tabelle 2**

| Bsp. | Ausgangsprodukt | Zielprodukt | | | |
|---|---|---|---|---|---|
| | | Struktur | Schmelzpunkt | Ausbeute | RG (HPLC) |
| 8 | 2-Oxethylmercapto-5-nitrobenzoesäure | 2-Oxethylsulfonyl-5-nitrobenzoesäure | 172 - 175°C | 95,8 % | 97,9 % |
| 9 | 3-Oxethylmercapto-4-nitrobenzoesäure | 3-Oxethylsulfonyl-4-nitrobenzoesäure | 175 - 177°C | 97,0 % | 98,2 % |
| 10 | 5-Oxethylmercapto-2-nitrobenzoesäure | 5-Oxethylsulfonyl-2-nitrobenzoesäure | 166 - 168°C | 96,6 % | 98,5 % |
| 11 | 3-Oxethylmercapto-2-nitrobenzoesäure | 3-Oxethylsulfonyl-2-nitrobenzoesäure | 179 - 181°C | 94,8 % | 98,5 % |
| 12 | 2-Oxethylmercapto-3-nitrobenzoesäure | 2-Oxethylsulfonyl-3-nitrobenzoesäure | 183 - 186°C | 91,5 % | 96,9 % |
| Die Analysen der Zielprodukte bestätigen die angegebenen Strukturen. | | | | | |

### Beispiel 13

In eine gerührte Suspension aus 201,5 Teilen 5-Chlor-2-nitrobenzoesäure und 250 Teilen Wasser werden langsam in ca. 40 Minuten 168 Teile Kaliumcarbonat eingetragen. Anschließend heizt man auf 55 bis 60°C, tropft in 2 Stunden 109 Teile Mercaptoethanol zu, rührt ca. 3 Stunden bei 70 bis 75°C nach, bis dünnschichtchromatografisch kein Ausgangsprodukt mehr nachweisbar ist, und stellt durch Zulauf von ca. 180 Teilen Eisessig einen pH-Wert von 1 ein.

Zu der entstandenen Suspension gibt man 2 Teile Natriumwolframat-dihydrat und tropft dann im Verlaufe von 60 Minuten bei von 60°C auf 90°C ansteigender Temperatur 350 Teile 30%iges wäßriges Wasserstoffperoxid zu und rührt 2 bis 3 Stunden bei 90°C nach (Kontrolle auf vollständige Umsetzung durch Dünnschichtchromatografie oder HPLC).

Anschließend kühlt man auf 0 bis 5°C ab, rührt 2 Stunden nach und saugt die ausgefallenen farblosen Kristalle ab. Nach Waschen mit Eiswasser bis zum neutralen Filtratablauf und Trocknen im Vakuum bei 90°C erhält man 260 Teile 5-Oxethylsulfonyl-2-nitrobenzoesäure der Formel 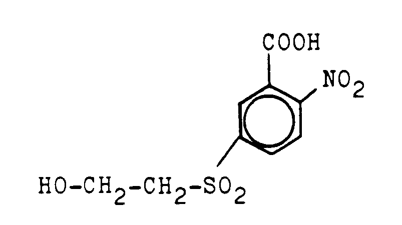
vom Schmelzpunkt 165 bis 167°C und einem Reingehalt (HPLC) von 97,9 %.

Ersetzt man die 5-Chlor-2-nitrobenzoesäure durch 4-Chlor-3-nitrobenzoesäure und arbeitet in der angegebenen Weise, so erhält man 258 Teile 4-Oxethylsulfonyl-3-nitrobenzoesäure vom Schmelzpunkt 190 bis 192°C und einem Reingehalt von 97,0 %.

### Beispiel 14

In 100 Teile Dimethylformamid werden unter Rühren nacheinander 40,3 Teile 3-Chlor-4-nitrobenzoesäure und 44,6 Teile Kaliumcarbonat eingetragen. Man heizt auf 40 bis 45°C und tropft dann in 45 Minuten 75 Teile 40%iges wäßriges Mercaptoethanol zu.

Nach Heizen auf 90 bis 95°C wird so lange nachgerührt, bis dünnschichtchromatografisch kein Ausgangsprodukt mehr nachweisbar ist, anschließend mit ca. 65 Teilen 30%iger Salzsäure auf pH 1,5 eingestellt und auf 50 bis 60°C abgekühlt.

Man gibt 1 Teile Wolframtrioxid zu, tropft im Verlaufe von 90 Minuten gleichmäßig 78,5 Teile 40%iges wäßriges Wasserstoffperoxid in das gerührte Reaktionsgemisch ein, wobei die Innentemperatur bis 95°C ansteigen darf, und rührt bis zur vollständigen Oxidation nach (ca. 5 Stunden, Kontrolle durch Dünnschichtchromatografie oder HPLC).

Nun setzt man 300 Teile Wasser zu, kühlt unter Rühren auf 0 bis 5°C ab, rührt 90 Minuten bei 0 bis 5°C nach und saugt dann den ausgefallenen farblosen Niederschlag ab. Nach Neutralwaschen mit Eiswasser und Trocknen bei 80°C im Vakuum erhält man 51 Teile 3-Oxethylsulfonyl-4-nitrobenzoesäure der Formel 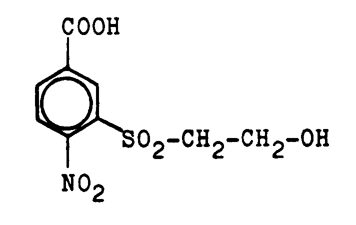
vom Schmelzpunkt 176 bis 177°C und einem Reingehalt (HPLC) von 99,3 %.

Ersetzt man die 3-Chlor-4-nitrobenzoesäure durch 3-Chlor-2-nitrobenzoesäure und arbeitet in der angegebenen Weise, so erhält man 48 Teile 3-Oxethylsulfonyl-2-nitrobenzoesäure vom Schmelzpunkt 178 bis 181°C und einem Reingehalt (HPLC) von 98,8 %.

### Beispiel 15

Zu einer auf 80 bis 85°C erwärmten Mischung aus 40 Teilen Eisenpulver und 200 Teilen Wasser gibt man innerhalb 30 Minuten unter Rühren gleichmäßig 75 Teile 3-Oxethylsulfonyl-2-nitrobenzoesäure und hält während des Eintragens die Reaktionstemperatur auf 80 bis 85°C. Nach beendeter Zugabe rührt man 30 Minuten nach, stellt mit wäßriger Sodalösung einen pH-Wert von 8,5 ein und klärt heiß vom ausgefallenen Eisenhydroxid. Der Filterrückstand wird zweimal mit wenig heißem Wasser gewaschen. Anschließend werden die vereinigten Filtrate mit 25 Teilen Eisessig angesäuert, im Vakuum auf 150 Volumenteile eingeengt und nachfolgend auf 0 bis 5°C kaltgerüht. Der ausgefallene schwach bräunlich gefärbte Niederschlag wird auf einer Nutsche isoliert, mit wenig Eiswasser abgedeckt und bei 60°C im Vakuum getrocknet. Man erhält 78 Teile 3-Oxethylsulfonyl-anthranilsäure-acetat der Formel 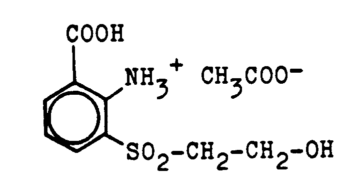
vom Schmelzpunkt 286 bis 289°C und einem Reingehalt (durch Diazotierung) von 99,5 %.

| | | | |
|---|---|---|---|
| Analyse: | C: | 43,2/43,3 % | (ber. 43,28 %); |
| | H: | 4,9/5,1 % | (ber. 4,92 %); |
| | N: | 4,6/4,6 % | (ber. 4,59 %); |
| | S: | 10,3/10,5 % | (ber. 10,49 %). |

### Beispiel 16

Ersetzt man im vorhergehenden Beispiel 15 die 3-Oxethylsulfonyl-2-nitrobenzoesäure durch 2-Oxethylsulfonyl-3-nitrobenzoesäure und stellt nach der Klärfiltration vom Eisenschlamm einen pH-Wert von 6,5 bis 7,0 ein, arbeitet aber sonst in der angegebenen Weise, so erhält man 64 Teile 2-Oxethylsulfonyl-3-aminobenzoesäure der Formel 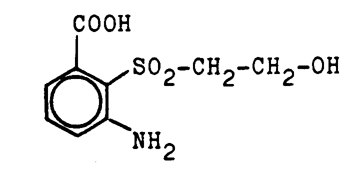
vom Schmelzpunkt 75 bis 77°C und einem Reingehalt (durch Diazotierung) von 99,1 %. Die Elementaranalyse belegt obige Struktur.

### Beispiel 17

In einem Hydrierautoklav werden 1200 Teile Wasser und 137,5 Teile 5-Oxethylsulfonyl-2-nitrobenzoesäure vorgelegt und 10 Teile Edelmetallkatalysator (5 % Palladium auf Kohle) zugegeben. Der Autoklav wird verschlossen und der Gasraum durch dreimaliges Spülen mit Stickstoff, dann mit Wasserstoff sauerstoff- und stickstoff-frei gemacht.

Anschließend werden 40 bar Wasserstoff aufgedrückt und auf 90°C aufgeheizt. Der Wasserstoffdruck wird durch stetiges Nachdrücken von Wasserstoff auf 40 bis 45 bar gehalten. Nach 2 Stunden bei 90°C bricht die Wasserstoffaufnahme ab. Der Autoklav-Inhalt wird heiß über ein Druckfilter vom Katalysator geklärt und das Filtrat auf 0 bis 5°C kaltgerührt. Die ausgefallenen farblosen Kristalle werden abgesaugt, mit wenig Eiswasser gewaschen und im Vakuum bei 60°C getrocknet.

Man erhält 108 Teile 5-Oxethylsulfonyl-anthranilsäure der Formel 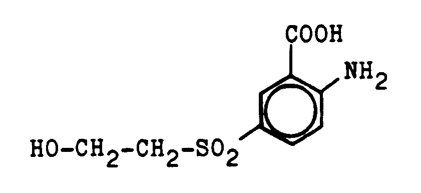
vom Schmelzpunkt 164 bis 167°C mit einem Reingehalt (bestimmt durch Diazotierung) von 99,9 %.

### Beispiel 18

Beispiel 17 wird exakt wiederholt, aber an Stelle der 1200 Teile Wasser wird die wäßrige Mutterlauge aus Beispiel 17 (ca. 1250 Teile) und an Stelle von Frischkontakt der aus der Hydrierung durch Klärfiltration abgetrennte Pd-Katalysator eingesetzt.

Man erhält 120 Teile 5-Oxethylsulfonyl-anthranilsäure vom Schmelzpunkt 164 bis 167°C und einem Reingehalt (Diazotierung) von 99,8 %.

### Beispiele 19 bis 27

Verfährt man weiter wie in den Beispielen 17 und 18, setzt jeweils 137,5 Teile 5-Oxethylsulfonyl-2-nitrobenzoesäure ein, dazu jeweils die wäßrige Mutterlauge und den Pd-Kontakt des vorhergehenden Ansatzes, so erhält man jeweils ca. 122 Teile 5-Oxethylsulfonyl-anthranilsäure vom Schmelzpunkt 164 bis 166°C und einem Reingehalt (Diazotierung) von > 99 %, d.h., Mutterlauge und Katalysator sind jeweils mindestens 10 mal ohne Produktverschlechterung oder Ausbeutesenkung einsetzbar.

### Beispiel 28

Ersetzt man in Beispiel 17 den Palladium-Katalysator durch einen handelsüblichen Platin- oder Nickel-Trägerkatalysator und arbeitet in der angegebenen Weise, so erhält man die 5-Oxethylsulfonyl-anthranilsäure in vergleichbarer Ausbeute und Qualität.

### Beispiele 29 bis 31

Ersetzt man in den vorangegangenen Beispielen 16 oder 17 bis 27 die dort eingesetzten Oxethylsulfonyl-nitrobenzoesäuren durch die in Tabelle 3 aufgeführten Ausgangsprodukte und arbeitet in der angegebenen Weise, so erhält man die entsprechenden Oxethylsulfonylaminobenzoesäuren in den ebenfalls in Tabelle 3 angeführten Ausbeuten und Qualitäten (Schmelzpunkt/Reingehalt durch Diazotierung).

**Tabelle 3**

| Bsp. | Ausgangsprodukt | Zielprodukt | | | |
|---|---|---|---|---|---|
| | | Struktur | Schmelzpunkt | Ausbeute | RG (Diaz.) |
| 29 | 4-Oxethylsulfonyl-3-nitrobenzoesäure | 4-Oxethylsulfonyl-3-aminobenzoesäure | 183 - 186°C | 97,5 % | 99,2 % |
| 30 | 2-Oxethylsulfonyl-5-nitrobenzoesäure | 2-Oxethylsulfonyl-5-aminobenzoesäure | 47 - 50°C | 91,8 % | 99,4 % |
| 31 | 3-Oxethylsulfonyl-4-nitrobenzoesäure | 3-Oxethylsulfonyl-4-aminobenzoesäure | 220 - 223°C | 97,1 % | 99,4 % |

## Patentansprüche

1. Verbindungen der allgemeinen Formel 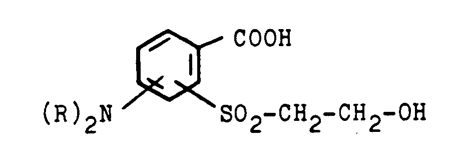 in welcher R ein Wasserstoff- oder Sauerstoffatom bedeutet und die Gruppe -N(R)₂ und die Oxethylsulfonylgruppe in ortho- oder para-Stellung zueinander stehen, ausgenommen die 5-Oxethylsulfonyl-2-aminobenzoesäure.

2. Verbindungen der allgemeinen Formel 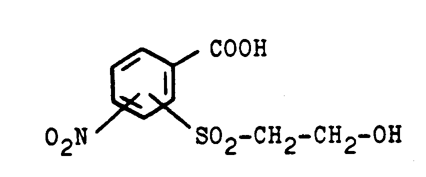 in welcher die Nitro- und Oxethylsulfonylgruppe in ortho- oder para-Stellung zueinander stehen.

3. Verbindungen der allgemeinen Formel 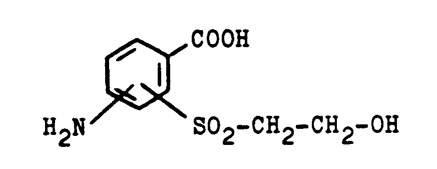 in welcher die Amino- und Oxethylsulfonylgruppe in ortho- oder para-Stellung zueinander stehen, ausgenommen die 5-Oxethylsulfonyl-2-aminobenzoesäure.

4. Verfahren zur Herstellung von Verbindungen der allgemeinen Formel (1) 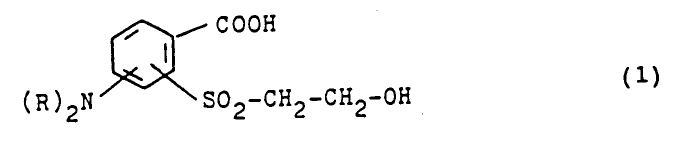 in welcher R ein Wasserstoff- oder Sauerstoffatom bedeutet und die Gruppe -N(R)₂ und die Oxethylsulfonylgruppe in ortho- oder para-Stellung zueinander stehen, dadurch gekennzeichnet, daß man 1 Mol einer Halogen-nitrobenzoesäure der allgemeinen Formel (2) 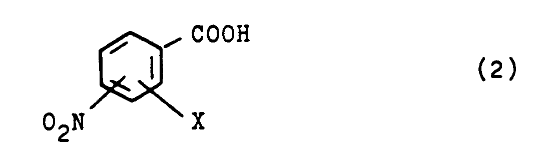 in welcher X ein Fluor-, Chlor-, Brom- oder Jodatom bedeutet und die Nitrogruppe und das Halogenatom in ortho- oder para-Stellung zueinander stehen, mit mindestens 1 Mol Mercaptoethanol in wäßriger Lösung oder Suspension oder in einem organischen Lösungsmittel in Gegenwart eines säurebindenden Mittels bei Temperaturen von 20 bis 100°C in die entsprechenden Oxethylmercapto-nitrobenzoesäuren der allgemeinen Formel (3) 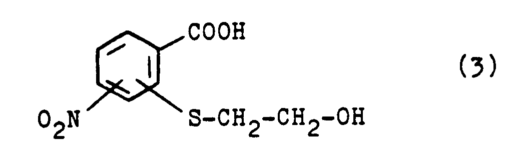 überführt, diese in an sich bekannter Weise zu den entsprechenden Oxethylsulfonyl-nitrobenzoesäuren der genannten Formel (1) (R = O) oxidiert und ggfs. letztere in an sich bekannter Weise zu den entsprechenden Oxethylsulfonyl-aminobenzoesäuren der allgemeinen Formel (4) 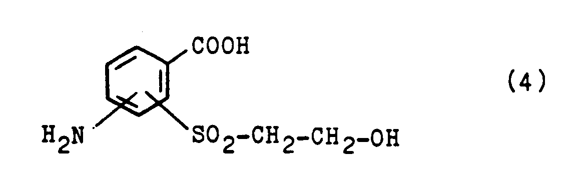 in welcher die Amino- und Oxethylsulfonylgruppe in ortho- oder para-Stellung zueinander stehen, reduziert.

5. Verfahren nach Anspruch 4, dadurch gekennzeichnet, daß man 1 Mol Halogennitrobenzoesäure mit 1,1 bis 1,8 Mol Mercaptoethanol bei Temperaturen von 35 bis 60°C umsetzt.

6. Verfahren nach mindestens einem der Ansprüche 4 und 5, dadurch gekennzeichnet, daß man die Umsetzung der Halogennitrobenzoesäure mit Mercaptoethanol in Gegenwart eines Alkalimetallhydroxids oder -carbonats als säurebindendes Mittel vornimmt.

7. Verfahren nach mindestens einem der Ansprüche 4 bis 6, dadurch gekennzeichnet, daß man die Oxidation der Verbindungen der in Anspruch 4 genannten allgemeinen Formel (3) mit Wasserstoffperoxid in Gegenwart einer Verbindung des 6-wertigen Wolframs bei pH-Werten < 7 bei Temperaturen zwischen etwa 30° und etwa 120°C durchführt.

8. Verfahren nach mindestens einem der Ansprüche 4 bis 7, dadurch gekennzeichnet, daß man die Reduktion der Verbindungen der in Anspruch 4 genannten allgemeinen Formel (1) (R = O) in wäßriger Lösung oder Suspension mit Eisen bei Temperaturen von etwa 70 bis etwa 120°C durchführt.

9. Verfahren nach mindestens einem der Ansprüche 4 bis 7, dadurch gekennzeichnet, daß man die Reduktion der Verbindungen der in Anspruch 4 genannten allgemeinen Formel (1) (R = O) in wäßriger Lösung oder Suspension mit katalytisch aktiviertem Wasserstoff in Gegenwart eines Nickel- oder Edelmetallkatalysators bei Temperaturen von etwa 70 bis etwa 120°C durchführt.

10. Verwendung der von Anspruch 1 umfaßten Oxethylsulfonyl-aminobenzoesäuren als Diazokomponenten zur Herstellung faserreaktiver Azofarbstoffe.

## Claims

1. A compound of the formula 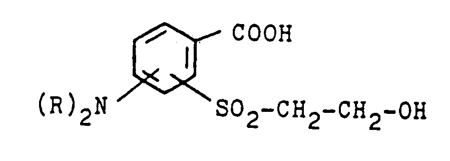 in which R denotes a hydrogen or oxygen atom and the -N(R)₂ group and the hydroxyethylsulfonyl group are in the ortho- or para-position relative to one another, with the exception of 5-hydroxyethylsulfonyl-2-aminobenzoic acid.

2. A compound of the formula 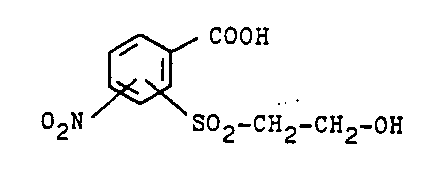 in which the nitro and hydroxyethylsulfonyl group are in the ortho- or para-position relative to one another.

3. A compound of the formula 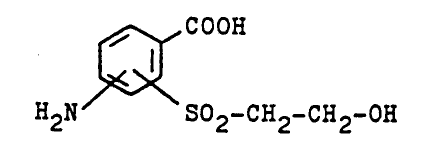 in which the amino and hydroxyethylsulfonyl group are in the ortho- or para-position relative to one another, with the exception of 5-hydroxyethylsulfonyl-2-aminobenzoic acid.

4. A process for the preparation of compounds of the formula (1) 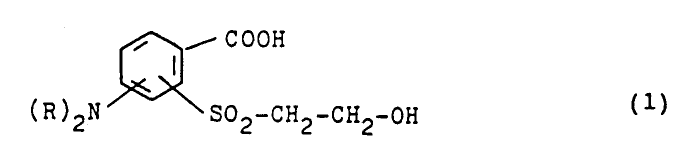 in which R denotes a hydrogen or oxygen atom and the -N(R)₂ group and the hydroxyethylsulfonyl group are in the ortho- or para-position relative to one another, which comprises converting 1 mol of a halonitrobenzoic acid of the formula (2) 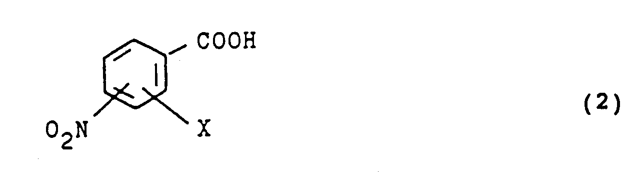 in which X denotes a fluorine, chlorine, bromine or iodine atom, and the nitro group and the halogen atom are in the ortho- or para-position relative to one another, with at least 1 mol of mercaptoethanol in an aqueous solution or suspension or in an organic solvent in the presence of an acid-binding agent at temperatures from 20 to 100°C, to the corresponding hydroxyethylmercaptonitrobenzoic acid of the formula (3) 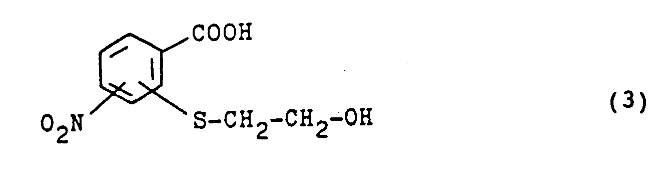 oxidizing these acids in a manner known per se to the corresponding hydroxyethylsulfonylnitrobenzoic acids of the formula (1) (R=O) mentioned and reducing the latter, if necessary, in a manner known per se to the corresponding hydroxyethylsulfonylaminobenzoic acids of the general formula (4) 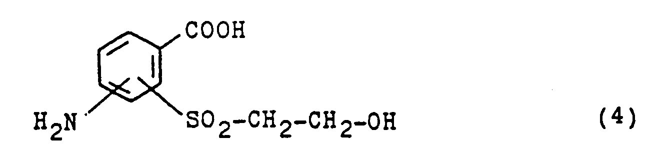 in which the amino and hydroxyethylsulfonyl group are in the ortho- or para-position relative to one another.

5. The process as claimed in claim 4, wherein 1 mol of halonitrobenzoic acid is reacted with 1.1 to 1.8 mol of mercaptoethanol at temperatures from 35 to 60°C.

6. The process as claimed in at least one of claims 4 and 5, wherein the reaction of the halonitrobenzoic acid with mercaptoethanol is carried out in the presence of an alkali metal hydroxide or alkali metal carbonate as the acid-binding agent.

7. The process as claimed in at least one of claims 4 to 6, wherein the oxidation of the compounds of the formula (3) mentioned in claim 4 is carried out using hydrogen peroxide in the presence of a compound of hexavalent tungsten at pH values of < 7 at temperatures between about 30° and about 120°C.

8. The process as claimed in at least one of claims 4 to 7, wherein the reduction of the compounds of the formula (1) (R=O) mentioned in claim 4 is carried out in an aqueous solution or suspension using iron at temperatures of about 70 to about 120°C.

9. The process as claimed in at least one of claims 4 to 7, wherein the reduction of the compounds of the formula (1) (R=O) mentioned in claim 4 is carried out in an aqueous solution or suspension using catalytically activated hydrogen in the presence of a nickel or noble metal catalyst at temperatures of about 70 to about 120°C.

10. Use of the hydroxyethylsulfonylaminobenzoic acids claimed in claim 1 as diazo components for the preparation of fiber-reactive azo dyes.

## Revendications

1. Composés répondant à la formule générale : 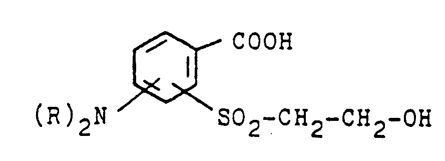 dans laquelle R désigne un atome d'hydrogène ou un atome d'oxygène et le groupe -N(R)₂ et le groupe oxéthylsulfonyle sont en position ortho ou para l'un par rapport à l'autre, à l'exception de l'acide 5-oxéthylsulfonyl-2-aminobenzoïque.

2. Composés répondant à la formule générale : 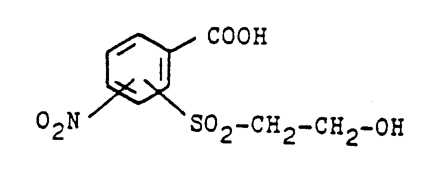 dans laquelle le groupe nitro et le groupe oxéthylsulfonyle sont en position ortho ou para l'un par rapport à l'autre.

3. Composés répondant à la formule générale : 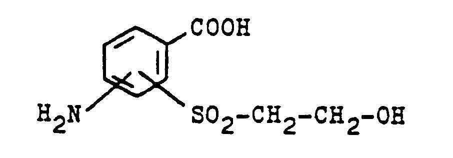 dans laquelle le groupe amino et le groupe oxéthylsulfonyle sont en position ortho ou para l'un par rapport à l'autre, à l'exception de l'acide 5-oxéthylsulfonyl-2-aminobenzoïque.

4. Procédé de préparation de composés répondant à la formule générale I : 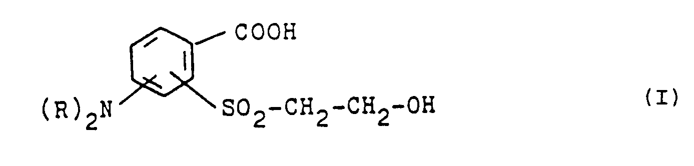 dans laquelle R désigne un atome d'hydrogène ou un atome d'oxygène et le groupe -N(R)₂ et le groupe oxéthylsulfonyle sont en position ortho ou para l'un par rapport à l'autre, caractérisé en ce que l'on transforme 1 mole d'un acide halogéno-nitrobenzoïque répondant à la formule générale II : 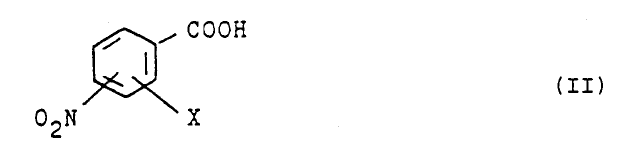 dans laquelle X désigne un atome de fluor, de chlore, de brome ou d'iode, et le groupe nitro et l'atome d'halogène sont en position ortho ou para l'un par rapport à l'autre, avec au moins 1 mole de mercaptoéthanol en solution ou en suspension aqueuse ou dans un solvant organique, en présence d'un agent fixant les acides, à des températures de 20 à 100 °C, en les acides oxéthylmercaptonitrobenzoïques correspondants répondant à la formule générale III : 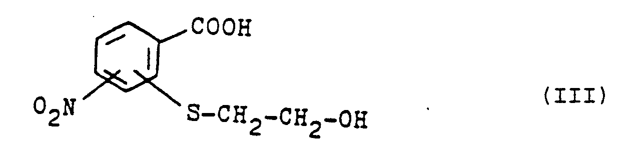 en ce qu'on oxyde ceux-ci d'une manière connue en soi en les acides oxéthylsulfonyl-nitrobenzoïques correspondants répondant à la formule I (R = 0), et le cas échéant en ce qu'on réduit ces derniers d'une manière connue en soi en les acides oxéthylsulfonyl-aminobenzoïques correspondants répondant à la formule IV : 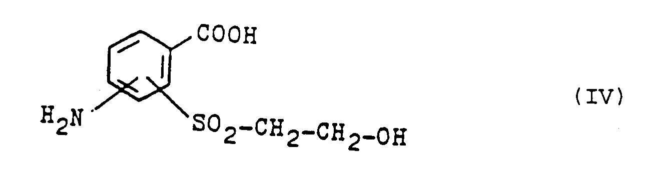 dans laquelle le groupe amino et le groupe oxéthylsulfonyle sont en position ortho ou para l'un par rapport à l'autre.

5. Procédé selon la revendication 4, caractérisé en ce que l'on fait réagir 1 mole d'acide halogéno-nitrobenzoïque avec 1,1 à 1,8 mole de mercaptoéthanol à des températures de 35 à 60 °C.

6. Procédé selon au moins une des revendications 4 et 5, caractérisé en ce que l'on effectue la réaction de l'acide halogéno-nitrobenzoïque avec le mercaptoéthanol en présence d'un hydroxyde ou carbonate de métal alcalin comme agent de fixation des acides.

7. Procédé selon au moins une des revendications 4 à 6, caractérisé en ce que l'on effectue l'oxydation des composés répondant à la formule générale III indiquée dans la revendication 4 avec du peroxyde d'hydrogène, en présence d'un composé du tungstène hexavalent, à des pH inférieurs à 7, à des températures comprises entre environ 30 °C et environ 120 °C.

8. Procédé selon au moins une des revendications 4 à 7, caractérisé en ce que l'on effectue la réduction des composés répondant à la formule générale I indiquée dans la revendication 4 (R = 0), en solution ou suspension aqueuse, avec du fer, à des températures d'environ 70 à environ 120 °C.

9. Procédé selon au moins une des revendications 4 à 7, caractérisé en ce que l'on effectue la réduction des composés répondant à la formule générale I indiquée dans la revendication 4 (R = 0) en solution ou suspension aqueuse, avec de l'hydrogène activé catalytiquement, en présence d'un catalyseur à base de nickel ou de métal précieux, à des températures d'environ 70 à environ 120 °C.

10. Utilisation des acides oxéthylsulfonyl-aminobenzoïques selon la revendication 1 comme constituants diazoïques pour la préparation de colorants azoïques réagissant avec les fibres.
